# EUROPÄISCHE PATENTSCHRIFT

(11) **EP 1 243 587 B1**
(45) Veröffentlichungstag und Bekanntmachung des Hinweises auf die Patenterteilung: **03.05.2006**
(21) Anmeldenummer: 02005261.9
(22) Anmeldetag: 11.03.2002
(51) Int. Cl.: C07D 315/00, C11B 9/00

(54) **Verfahren zur Herstellung von 15-Pentadecanolid**
Process for the preparation of 15-pentadecanolide
Procédé de préparation de 15-pentadécanolide

(30) Priorität: 22.03.2001 DE 10113963
(43) Veröffentlichungstag der Anmeldung: 25.09.2002
(73) Patentinhaber: Symrise GmbH & Co. KG, 37603 Holzminden (DE)
(72) Erfinder: Kuhn, Walter, Dr., 37603 Holzminden (DE); Koch, Oskar, Dr., 37079 Göttingen (DE); Funk, Hans-Ulrich, 37697 Lauenförde (DE); Senft, Gerhard, 37603 Holzminden (DE)
(74) Vertreter: Eisenführ, Speiser & Partner

(56) Entgegenhaltungen:
- YU N. OGIBIN: "RING-EXPANSION REACTION OF 1-HYDROPEROXY-16-OXABICYCLO[10.4.0]HEXADEC ANE CATALYSED BY COPPER IONS:" RUSSIAN CHEMICAL BULLETIN., Bd. 47, Nr. 6, Juni 1998 (1998-06), Seiten 1166-9, XP002201951 PLENUM PUBLISHING CO, NEW YORK, NY., US ISSN: 1066-5285

## Beschreibung

Die Erfindung betrifft ein Verfahren zur Herstellung von 15-Pentadecanolid durch Hydrierung von 15-Pentadecenolid und seine Verwendung.

15-Pentadecanolid (Oxacyclohexadecan-2-on) ist ein wertvoller Moschusriechstoff, der im Angelikawurzelöl vorkommt. (K. Bauer, A. Garbe, Common Fragrance and Flavor Materials, S. 104, VCH, Weinheim, 1985).

Es ist bekannt, 15-Pentadecanolid durch Hydrierung der Doppelbindung von 15-Pentadecenolid herzustellen.

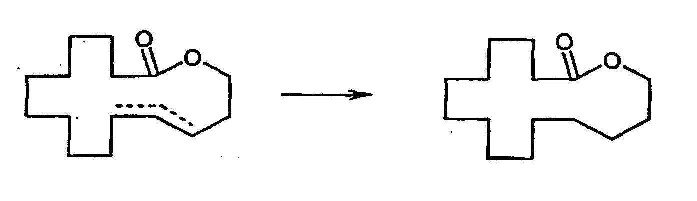

15-Pentadecenolid kann gemäß Russian Chemical Bulletin, 47, 6, 1998, 1166 hergestellt werden und liegt üblicherweise als Gemisch von 15-Pentadecen-11-olid und 15-Pentadecen-12-olid vor. Dieses Doppelbindungsisomerengemisch wird im vorliegenden Falle mit 15-Pentadecen-11(12)-olid bezeichnet.

In JP 01090182 A2 (Chemical Abstract Number 1989 : 534017) wird als Katalysator Raney-Nickel zur Hydrierung von 15-Pentadecen-11(12)-olid in ethanolischer Lösung eingesetzt.

In Russian Chemical Bulletin, 47, 6, 1998, 1166 sind Palladium-Katalysatoren zur Hydrierung beschrieben. 15-Pentadecen-11(12)-olid wird mit Palladium auf Aktivkohle oder Palladium auf Aluminiumoxid zu 15-Pentadecanolid hydriert, gegebenenfalls unter Zusatz einer Base wie Natriumcarbonat.

Die erwähnten Verfahren zur Herstellung von 15-Pentadecanolid aus 15-Pentadecen-11(12)-olid zeigen bei der großtechnischen Produktion gravierende Nachteile. Neben den hohen Wasserstoffdrücken und eventuellen Basenzusätzen sind insbesondere die Verdünnung mit Lösemittel und die hohe Einsatzmenge an Katalysator nachteilig. Im Falle von Raney-Nickel sind die pyrophoren, toxischen und allergenen Eignenschaften des Katalysators besonders nachteilig

Im Allgemeinen weist das aus diesen Hydrierungen stammende 15-Pentadecanolid nach der thermischen Belastung einer Destillation geruchlich eine brandige Note auf, was oftmals eine weitere Reinigung erfordert, um zu einer parfümistisch akzeptablen Qualität zu gelangen.

Es besteht weiterhin der Bedarf ein Verfahren zu finden, das 15-Pentadecanolid wirtschaftlich, in hoher Ausbeute und guter parfümistischer Qualität liefert.

Die Erfindung betrifft ein Verfahren zur Herstellung von 15-Pentadecanolid durch Hydrierung von 15-Pentadecen-11(12)-olid in Gegenwart eines Katalysators, enthaltend mindestens ein Metall der 8. Nebengruppe in elementarer Form, welches gegebenenfalls auf einen Träger aufgebracht ist, wobei das Gewichtsverhältnis von Metall zu 15-Pentadecen-11(12)-olid unterhalb von 1:20 000 liegt und als Metall der 8. Nebengruppe Palladium, Platin, Ruthenium oder Rhodium eingesetzt wird.

Die Säurezahl entspricht der Anzahl der Milligramm an Kaliumhydroxid, die zur Neutralisation der freien organischen Säuren eines Gramms des Hydrierungsrohproduktes verbraucht werden.

Unter dem Hydrierungsrohprodukt wird das direkt aus der Hydrierung stammende Produkt verstanden, d.h. das ohne weitere Aufreinigung vorliegende, rohe 15-Pentadecanolid.

Das so hergestellte 15-Pentadecanolid besitzt schon als Hydrierungsrohprodukt parfümistische Qualität und bedarf keiner weiteren Reinigung. Gegebenenfalls kann eine weitere Aufreinigung durchgeführt werden. Die Ausbeute ist nahezu quantitativ (Ausbeute an 15-Pentadecanolid größer 98,5%). Bevorzugterweise wird die Hydrierung lösemittelfrei durchgeführt. Das erfindungsgemäße Verfahren erlaubt eine Hydrierung unter milden Bedingungen.

Die Metalle der 8. Nebengruppe können beispielsweise in fein verteilter Form, aufgebracht auf Träger oder zusammen mit anderen Metallen eingesetzt werden (z.B. Mischungen, Legierungen). Die Katalysatoren können Dotierungen mit einem oder mehreren beliebigen Metallen enthalten.

Die erfindungsgemäßen Metalle können auf organische oder anorganische Trägermaterialien aufgebracht sein. Die Katalysatoren können ein Trägermaterial oder Mischungen von Trägermaterialien enthalten. Als vorteilhafte Trägermaterialen seien genannt Aktivkohle, Kohle, Aluminiumoxide, Metalloxide, Kieselgele, Zeolithe, Tone, Tongranulate, amorphe Aluminiumsilicate, oder sonstige anorganische Träger. Ein bevorzugtes Trägermaterial ist Aktivkohle.

Ein ganz besonders bevorzugter Katalysator ist Palladium auf Aktivkohle.

In dem erfindungsgemäßen Verfahren werden Katalysatormengen eingesetzt, bei denen das Gewichtsverhältnis von Metallmenge zu 15-Pentadecen-11(12)-olid unterhalb von 1:20 000 liegt, bevorzugt im Bereich 1:50 000 bis 1:10 000 000, insbesondere bevorzugt im Bereich 1:100 000 bis 1:5 000 000 und ganz besonders bevorzugt im Bereich 1:250 000 bis 1:1 000 000.

Die Metallmenge bezieht sich dabei auf den absoluten Gehalt des Metalls der 8. Nebengruppe oder den Gesamtgehalt an Metallen der 8. Nebengruppe, d.h. ohne Trägermaterial und ohne eventuell vorhandenes Wasser oder Verdünnungsmittel.

Werden Trägermaterialien enthaltende Katalysatoren verwendet, kann der Anteil an Metall auf dem Trägermaterial im allgemeinen 0,5 bis 50 Gew.%, bevorzugt 1 bis 20 Gew.%, insbesondere bevorzugt 3 bis 10 Gew.%, bezogen auf den trockenen Katalysator, betragen.

Für das erfindungsgemäße Verfahren kann der Katalysator im trockenen oder feuchten Zustand (Restfeuchte an Wasser) verwendet werden.

Das erfindungsgemäße Verfahren kann in Gegenwart von Lösemitteln durchgeführt werden, bevorzugt ist die lösemittelfreie Hydrierung.

Die Hydrierung kann bei Temperaturen von 0 bis 150°C durchgeführt werden. Geeignet sind Temperaturen im Bereich von 20 bis 100°C, bevorzugt im Bereich von 30 bis 90°C, insbesondere bevorzugt bei 45 bis 80°C. Ganz besonders bevorzugt ist der Temperaturbereich von 60 bis 75°C.

Erfindungsgemäß werden die Hydrierungen mit elementarem Wasserstoff durchgeführt.

Der Wasserstoffdruck liegt geeigneterweise bei 1 bis 100 bar, bevorzugt bei 1 bis 30 bar, insbesondere bevorzugt bei 5 bis 20 bar.

Die Reaktionszeit beträgt bevorzugt 1 bis 100 Stunden, insbesondere bevorzugt 10 bis 50 Stunden.

Die Säurezahl des Hydrierungsrohproduktes (rohes 15-Pentadecanolid) weist Säurezahlen von maximal 1 mg Kaliumhydroxid/g Produkt auf.

Das nach dem erfindungsgemäßen Verfahren hergestellte 15-Pentadecanolid kann ohne weitere Aufreinigungsschritte eingesetzt werden, insbesondere weist es eine gute parfümistische Qualität auf.

Das nach dem erfindungsgemäßen Verfahren hergestellte 15-Pentadecanolid kann insbesondere verwendet werden als Riechstoff, in Parfümkompositionen, Parfümölen oder Duftkompositionen.

Ein weiteres Verwendungsgebiet sind Hygiene- oder Pflegeprodukte, insbesondere im Bereich des Haushaltes und der Körperpflege.

Die das nach dem erfindungsgemäßen Verfahren hergestellte 15-Pentadecanolid enthaltenden Parfümöle können in konzentrierter Form, in Lösungen oder in sonstiger modifizierter Form verwendet werden für die Herstellung von z.B. Parfüm-Extraits, Eau de Parfums, Eau de Toilettes, Rasierwässer, Eau de Colognes, Preshave-Produkte, Splash-Colognes und parfümierten Erfrischungstüchern sowie die Parfümierung von sauren, alkalischen und neutralen Reinigungsmitteln, wie z.B. Fußbodenreinigern, Fensterglasreinigern, Geschirrspülmittel, Bad- und Sanitärreinigern, Scheuermilch, festen und flüssigen WC-Reinigern, pulver- und schaumformigen Teppichreinigern, flüssigen Waschmitteln, pulverförmigen Waschmitteln, Wäschevorbehandlungsmitteln wie Bleichmittel, Einweichmittel und Fleckenentfernen, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln wie z.B. festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ wie z.B. Hautcremes- und -lotionen, Gesichtscremes und -lotionen, Sonnenschutzeremes- und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten wie z.B. Haarsprays, Haargelen, Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien wie z.B. Achselsprays, Roll-ons, Deosticks, Deocremes oder Produkten der dekorativen Kosmetik.

Folgende Beispiele erläutern die Erfindung.

### Beispiel 1

### In Beispiel 1 liegt das Verhältnis von Pd:15-Pentadecen-11(12)-olid bei 1:281 667

In einem 4 m³ Rührautoklaven mit Begasungsrührer wurden 2 535 kg 15-Pentadecen-11(12)-olid (GC-Gehalt an 15-Pentadecen-11(12)-olid-Isomeren : 93,2 %; GC-Gehalt an 15-Pentadecanolid 2,9 %) und 300 g Palladium auf Aktivkohle (Pd-Gehalt: 5 Gew. %) mit 40 % Wassergehalt vorgelegt. Es wurde 12 Stunden bei 68-70°C und 20 bar Wasserstoffdruck hydriert. Nach Filtration des Hydrierungsrohproduktes wurden 2 548 kg 15-Pentadecanolid in 95,4 % Reinheit erhalten. Die Ausbeute lag bei >99%. Der Schmelzpunkt betrug 31°C. Die Säurezahl des Hydrierungsrohproduktes lag bei 0,6. Die so erhaltene Ware hat eine gute parfümistische Qualität.

### Beispiel 2

### In Beispiel 2 liegt das Verhältnis von Pd:15-Pentadecen-11(12)-olid bei 1:845 000

In einem 4 m³ Rührautoklaven mit Begasungsrührer wurden 2 535 kg 15-Pentadecen-11(12)-olid (GC-Gehalt: siehe Beispiel 1) und 100 g Palladium auf Aktivkohle (Pd-Gehalt: 5 Gew.-%) mit 40 % Wassergehalt vorgelegt. Es wurde 44 Stunden bei 68-69°C und 20 bar Wasserstoffdruck hydriert. Nach Filtration des Hydrierungsrohproduktes wurde 15-Pentadecanolid in 95,8 % Reinheit erhalten. Die Ausbeute lag bei >99 %. Der Schmelzpunkt betrug 31°C. Die Säurezahl des Hydrierungsrohproduktes lag bei 0,7. Die so erhaltene Ware hat eine gute parfümistische Qualität.

## Patentansprüche

1. Verfahren zur Herstellung von 15-Pentadecanolid durch Hydrierung von 15-Pentadecen-11(12)-olid in Gegenwart eines Katalysators, enthaltend mindestens ein Metall der 8. Nebengruppe in elementarer Form, welches gegebenenfalls auf einen Träger aufgebracht ist, wobei das Gewichtsverhältnis von Metall zu 15-Pentadecen-11(12)-olid unterhalb von 1:20 000 liegt und als Metall der 8. Nebengruppe Palladium, Platin, Ruthenium oder Rhodium eingesetzt wird.

2. Verfahren nach Anspruch 1, **dadurch gekennzeichnet, dass** als Metall Palladium verwendet wird.

3. Verfahren nach mindestens einem der Ansprüche 1 oder 2, **dadurch gekennzeichnet, dass** der Katalysator mindestens ein anorganisches Trägermaterial enthält.

4. Verfahren nach mindestens einem der Ansprüche 1 bis 3 **dadurch gekennzeichnet, dass** als Katalysator Palladium auf Aktivkohle eingesetzt wird.

5. Verfahren nach mindestens einem der Ansprüche 1 bis 4 **dadurch gekennzeichnet, dass** das Gewichtsverhältnis von Metallmenge zu 15-Pentadecen-11(12)-olid im Bereich 1:50 000 bis 1:10 000 000 liegt.

6. Verfahren nach mindestens einem der Ansprüche 1 bis 5 **dadurch gekennzeichnet, dass** die Hydrierung bei Temperaturen im Bereich von 20 bis 100°C durchgeführt wird.

7. Verfahren nach mindestens einem der Ansprüche 1 bis 6 **dadurch gekennzeichnet, dass** die Hydrierung lösernittelfrei durchgeführt wird.

8. Verfahren nach mindestens einem der Ansprüche 1 bis 7 **dadurch gekennzeichnet, dass** der Wasserstoffdruck bei 1 bis 100 bar liegt.

9. Verfahren nach mindestens einem der Ansprüche 1 bis 8 **dadurch gekennzeichnet, dass** das Hydrierungsrohprodukt nicht aufgereinigt wird.

## Claims

1. Process for the preparation of 15-pentadecanolide by hydrogenating 15-pentadecen-11(12)-olide in the presence of a catalyst containing at least one metal of subgroup 8 in elemental form, which is optionally applied on to a support, wherein the weight ratio of metal to 15-pentadecen-11(12)-olide is below 1:20 000 and palladium, platinum, ruthenium or rhodium is used as the metal of subgroup 8.

2. Process according to claim 1, **characterised in that** the metal used is palladium.

3. Process according to at least one of claims 1 or 2, **characterised in that** the catalyst contains at least one inorganic support material.

4. Process according to at least one of claims 1 to 3, **characterised in that** the catalyst used is palladium on activated carbon.

5. Process according to at least one of claims 1 to 4, **characterised in that** the weight ratio of the quantity of metal to 15-pentadecen-11(12)-olide is in the range of 1:50 000 to 1:10 000 000.

6. Process according to at least one of claims 1 to 5, **characterised in that** the hydrogenation is carried out at temperatures in the range from 20 to 100°C.

7. Process according to at least one of claims 1 to 6, **characterised in that** the hydrogenation is carried out without a solvent.

8. Process according to at least one of claims 1 to 7, **characterised in that** the hydrogen pressure is 1 to 100 bar.

9. Pocess according to at least one of claims 1 to 8, **characterised in that** the hydrogenation crude product is not purified.

## Revendications

1. Procédé de préparation du 15-pentadécanolide par hydrogénation du 15-pentadécen-11(12)-olide en présence d'un catalyseur contenant au moins un métal du 8^{ème} sous-groupe sous forme élémentaire, qui est éventuellement appliqué sur un support, où le rapport massique du métal au 15-pentadécen-11(12)-olide est inférieur à 1 : 20 000 et le palladium, le platine, le ruthénium ou le rhodium est utilisé comme métal du 8^{ème} sous-groupe.

2. Procédé selon la revendication 1 **caractérisé en ce que** le palladium est utilisé comme métal.

3. Procédé selon au moins l'une des revendications 1 ou 2 **caractérisé en ce que** le catalyseur contient au moins un support inorganique.

4. Procédé selon au moins l'une des revendications 1 à 3 **caractérisé en ce que** le palladium sur charbon actif est utilisé comme catalyseur.

5. Procédé selon au moins l'une des revendications 1 à 4 **caractérisé en ce que** le rapport massique de la quantité de métal au 15-pentadécen-11(12)-olide est situé dans le domaine de 1 : 50 000 à 1 : 10 000 000.

6. Procédé selon au moins l'une des revendications 1 à 5 **caractérisé en ce que** l'hydrogénation est conduite à des températures dans le domaine de 20 à 100°C.

7. Procédé selon au moins l'une des revendications 1 à 6 **caractérisé en ce que** l'hydrogénation est conduite sans solvant.

8. Procédé selon au moins l'une des revendications 1 à 7 **caractérisé en ce que** la pression d'hydrogène est située à 1 à 100 bar.

9. Procédé selon au moins l'une des revendications 1 à 8 **caractérisé en ce que** le produit brut d'hydrogénation n'est pas purifié.
